# EUROPEAN PATENT APPLICATION

(11) **EP 4 563 186 A1**
(43) Date of publication of application: **04.06.2025**
(21) Application number: 23845131.4
(22) Date of filing: 16.06.2023
(51) Int. Cl.: A61M 60/122

(54) **DRIVING APPARATUS AND BLOOD PUMP**

(30) Priority: 26.07.2022 CN 202210887639
(71) Applicant: Shenzhen Core Medical Technology Co., Ltd., Shenzhen, Guangdong 518000 (CN)
(72) Inventor: LOU, Sha, Shenzhen, Guangdong 518000 (CN); YU, Shunzhou, Shenzhen, Guangdong 518000 (CN)
(74) Representative: Murgitroyd & Company
(86) International application number: PCT/CN2023/100612
(87) International publication number: WO 2024/021918

(57) **Abstract**

Provided are a driving apparatus and a blood pump. A housing assembly (100) of the driving apparatus (10) has a through hole (101). A rotating shaft (200) has a shaft portion (210) and a ball head portion (220) fixedly connected to one end of the shaft portion (210). The end of the shaft portion (210) that goes away from the ball head portion (220) rotatably penetrates through the through hole (101). A first shaft sleeve (500) is mounted on the housing assembly (100). The first shaft sleeve (500) has an accommodating cavity (501) and a shaft hole (502). The accommodating cavity (501) fits the ball head portion (200). The shaft hole (502) and the accommodating cavity (501) communicate. The ball head portion (220) is rotatably arranged inside the accommodating cavity (501). The shaft portion (210) rotatably penetrates through the shaft hole (502). The diameter of the shaft hole (502) is smaller than the diameter of the ball head portion (220), so that the ball head portion (220) is confined to the accommodating cavity (501).

## Description

### CROSS-REFERENCE TO RELATED APPLICATIONS

This application claims priority of Chinese Patent Application No. CN 202210887639.0, filed on July 26, 2022, the entire content of which is incorporated herein by reference in its entirety.

### TECHNICAL FIELD

The present application relates to the technical field of medical devices, and in particular to a driving device and a blood pump.

### BACKGROUND

Blood pump is designed to be inserted percutaneously into a patient's blood vessel, such as an artery or a vein in a thigh or an axilla, and can be advanced into the patient's heart to function as a left ventricular assist device or a right ventricular assist device.

The blood pump generally includes a driving device and an impeller. The impeller is connected to a rotating shaft of the driving device. In order to achieve stable rotation of the rotating shaft and simultaneously ensure that the rotating shaft rotates with low friction, it is usually necessary to add a component for limiting the rotating shaft. However, currently many components are used for such limiting, which leads to a complex structure of the driving device and relatively great difficulty in assembly.

### SUMMARY

Accordingly, the present application provides a driving device and a blood pump with relatively low assembly difficulty.

According to a first aspect, the present application provides a driving device, which includes:
a housing assembly provided with a through hole;
a rotating shaft including a shaft portion and a spherical head portion fixedly connected to an end of the shaft portion, an end of the shaft portion away from the spherical head portion rotatably extending through the through hole; and
a first shaft sleeve mounted to the housing assembly, wherein the first shaft sleeve is provided with a receiving cavity and a shaft hole; the receiving cavity is adapted to the spherical head portion; the shaft hole is in communication with the receiving cavity; the spherical head portion is rotatably received in the receiving cavity; the shaft portion rotatably extends through the shaft hole; and a diameter of the shaft hole is less than a diameter of the spherical head portion, so as to limit the spherical head portion in the receiving cavity.

According to a second aspect, the present application provides a blood pump including an impeller and a driving device. The driving device includes:
a housing assembly provided with a through hole;
a rotating shaft including a shaft portion and a spherical head portion fixedly connected to an end of the shaft portion, an end of the shaft portion away from the spherical head portion rotatably extending through the through hole; and
a first shaft sleeve mounted to the housing assembly, wherein the first shaft sleeve is provided with a receiving cavity and a shaft hole; the receiving cavity is adapted to the spherical head portion; the shaft hole is in communication with the receiving cavity; the spherical head portion is rotatably received in the receiving cavity; the shaft portion rotatably extends through the shaft hole; a diameter of the shaft hole is less than a diameter of the spherical head portion, so as to limit the spherical head portion in the receiving cavity; and the end of the shaft portion away from the spherical head portion is connected to the impeller.

Details of one or more embodiments of the present application are set forth in the following drawings and descriptions. Other features, objects and advantages of the present application will become apparent with reference to the specification, drawings, and claims.

### BRIEF DESCRIPTION OF THE DRAWINGS

In order to illustrate technical solutions in the embodiments of the present application more clearly, the drawings used in the description of the embodiments or the prior art will be described briefly below. Apparently, the following described drawings are merely for some of the embodiments of the present application, and other drawings can be derived by those of ordinary skill in the art without any creative effort.
FIG. 1 is a perspective view of a blood pump according to an embodiment of the present application.
FIG. 2 is an exploded view of the blood pump of FIG. 1.
FIG. 3 is a cross-sectional view of the blood pump of FIG. 1 with a portion of a catheter omitted.
FIG. 4 is a perspective view of a rotor of the driving device of FIG. 3.
FIG. 5 is a perspective view of a flywheel of the rotor of FIG. 4.
FIG. 6 is a perspective view illustrating a stator and a magnetic conduction member of the driving device in FIG. 3 being assembled together.
FIG. 7 is a cross-sectional view of a rotating shaft and a first shaft sleeve being assembled together according to an embodiment of the present application.
FIG. 8 is a partial enlarged view of a portion I in FIG. 7.
FIG. 9 is a schematic view of assembling a rotating shaft, a first shaft sleeve and a second shaft sleeve according to an embodiment of the present application.
FIG. 10 is a perspective view of a first fixing portion of the first shaft sleeve in FIG. 9.
FIG. 11 is a schematic view of assembling a rotating shaft, a first shaft sleeve and a second shaft sleeve according to another embodiment of the present application.
FIG. 12 is a perspective view of a first fixing portion of the first shaft sleeve in FIG. 11.
FIG. 13 is a perspective view of a second fixing portion of the first shaft sleeve in FIG. 11.

### Description of reference signs:

1. Blood pump; 10. Driving device; 20. Impeller; 30. Cannula; 31. Blood inlet; 32. Blood outlet; 40. Catheter;
100. Housing assembly; 101. Through hole; 102. Inner cavity; 110. Outer housing; 120. Second shaft sleeve;
200. Rotating shaft; 210. Shaft portion; 211. Connecting end; 220. Spherical head portion;
300. Rotor; 301. First rotor unit; 302. Second rotor unit; 310. Flywheel; 311. Disc-shaped portion; 312. Inner tube; 313. Outer tube; 314. Annular cavity; 320. Magnet;
400. Stator; 401. First stator unit; 402. Second stator unit; 410. Magnetic core; 420. Coil;
500. First shaft sleeve; 501. Receiving cavity; 502. Shaft hole; 503. Flushing liquid hole; 504. First opening; 505. Second opening; 506. Third opening; 510. First fixing portion; 511. First half-hole; 512. First hemispherical groove; 513. First semi-liquid groove; 520. Second fixing portion; 521. Second half-hole; 522. Second hemispherical groove; 523. Second semi-liquid groove;
600. Magnetic conduction member; 610. Magnetic conduction plate.

### DETAILED DESCRIPTION OF THE EMBODIMENTS

In order to make the objectives, technical solutions, and advantages of the present application more clearly understood, the present application is described in further detail below in conjunction with the accompanying drawings and embodiments. It should be understood that specific embodiments described herein are intended only to interpret the present application and not intended to limit the present application.

It should be noted that when one element is referred to as "fixed to" or "arranged on" another element, it may be directly disposed on the other element or an intermediate element may exist. When one element is considered to be "connected to" another element, it may be directly connected to the other element or indirectly connected to the another element.

In addition, the terms "first" and "second" are used for descriptive purposes only, which cannot be construed as indicating or implying a relative importance, or implicitly specifying the number of the indicated technical features. Thus, the features defined with "first" and "second" may explicitly or implicitly include one or more features. In the description of the present application, "a plurality of" means two or more, unless specifically stated otherwise.

In the field of interventional medicine, an end of a device adjacent to an operator is usually defined as a proximal end, and an end of the device away from the operator is defined as a distal end.

A blood pump 1 and a driving device 10 according to embodiments of the present application are now described.

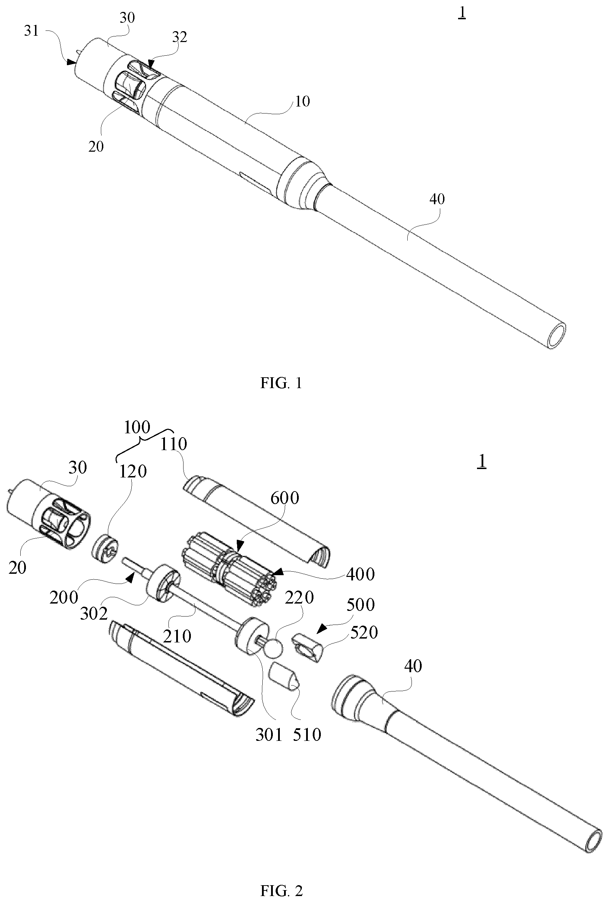

Referring to FIG. 1 to FIG. 3, the blood pump 1 includes a driving device 10 and an impeller 20. The driving device 10 is in transmission connection with the impeller 20, and the driving device 10 is capable of driving the impeller 20 to rotate.

Specifically, the blood pump 1 further includes a cannula 30 fixedly connected to a distal end of the driving device 10. The impeller 20 is rotatably accommodated in the cannula 30. The cannula 30 has a blood inlet 31 and a blood outlet 32. When the impeller 20 rotates, blood flows from the blood inlet 31 into the cannula 30 and then flows out of the blood outlet 32. In an embodiment, the cannula 30 extends through a heart valve, such as an aortic valve. The blood inlet 31 is located within a heart, and the blood outlet 32 and the driving device 10 are located in a blood vessel, such as an aorta outside a heart.

Specifically, the blood pump 1 further includes a catheter 40 connected to a proximal end of the driving device 10. The catheter 40 is configured to accommodate various supply lines. For example, the supply lines include a wire configured to be electrically connected to the driving device 10 and a cleaning pipeline configured to supply flushing fluid to the driving device 10 of the blood pump 1. Optionally, the flushing fluid is physiological saline, physiological saline containing heparin, or glucose, etc.

The driving device 10 includes a housing assembly 100, a rotating shaft 200, a rotor 300, a stator 400, and a first shaft sleeve 500.

A distal end of the housing assembly 100 is fixedly connected to the cannula 30, and a proximal end of the housing assembly 100 is fixedly connected to the catheter 40. An end of the housing assembly 100 is provided with a through hole 101, and the through hole 101 is located at an end of the housing assembly 100 adjacent to the sleeve 30. The housing assembly 100 further has an inner cavity 102. The inner cavity 102 is in communication with the through hole 101. The flushing liquid in the cleaning pipeline can flow into the inner cavity 102 and flow out of the housing assembly 100 from through hole 101.

Specifically, the housing assembly 100 includes an outer housing 110 and a second shaft sleeve 120.

The outer housing 110 is substantially a cylindrical housing with openings at both ends thereof. A distal end of the outer housing 110 is fixedly connected to the cannula 30, and a proximal end of the outer housing 110 is fixedly connected to the catheter 40. The outer housing 110 has the inner cavity 102. In some embodiments, the outer housing 110 is formed by splicing two substantially symmetrical half housings, so as to facilitate mounting the rotor 300, the stator 400, etc., in the inner cavity 102 of the outer housing 110.

The second shaft sleeve 120 is fixed in the outer housing 110. The through hole 101 is formed on the second shaft sleeve 120. The second shaft sleeve 120 is provided at an opening of an end of the outer housing 110 adjacent the cannula 30.

The rotating shaft 200 is rotatably mounted to the housing assembly 100. The rotating shaft 200 is fixedly connected to the impeller 20. The rotating shaft 200 can drive the impeller 20 to rotate. The rotating shaft 200 has a shaft portion 210 and a spherical head portion 220 fixedly connected to one end of the shaft portion 210. An end of the shaft portion 210 away from the spherical head portion 220 rotatably extends through the through hole 101. The end of the shaft portion 210 away from the spherical head portion 220 is fixedly connected to the impeller 20.

The shaft portion 210 is elongated. The shaft portion 210 has a connecting end 211 configured to be connected to the impeller 20, that is, the connecting end 211 is the end of the shaft portion 210 away from the spherical head portion 220. The shaft portion 210 is located partially in the outer housing 110, and partially out of the outer housing 110 or in the cannula 30. The connecting end 211 extends out of the outer housing 110 and is fixedly connected to the impeller 20. In the illustrated embodiment, the shaft portion 210 extends substantially along an axial direction of the outer housing 110, or an axis of the shaft portion 210 extends in a direction substantially coinciding with the axial direction of the outer housing 110.

The spherical head portion 220 is substantially spherical. The spherical portion 220 is fixedly connected to an end of the shaft portion 210 away from the connecting end 211. Specifically, the axis of the shaft portion 210 passes through a center of a sphere of the spherical head portion 220. In the illustrated embodiment, a diameter of the spherical head portion 220 is greater than a diameter of the shaft portion 210.

In some embodiments, the shaft portion 210 and the spherical head portion 220 are made of ceramic materials. Compared with metal materials, ceramics have higher processing precision, higher biocompatibility and mechanical strength, and better wear resistance and corrosion resistance. In some embodiments, the shaft portion 210 and the spherical head portion 220 are integrally formed. In some embodiments, the shaft portion 210 and the spherical head portion 220 may be fixedly connected together by assembling, welding, or adhering. In some embodiments, the spherical head portion 220 has a ball body and a diamond coating provided on a surface of the ball body, so that a surface of the spherical head portion 220 is smooth and has high wear resistance. In this case, a material of the ball body may have a certain rigidity, for example, may be metal, ceramic, etc., and the material of the ball body may be the same as that of the shaft portion 210.

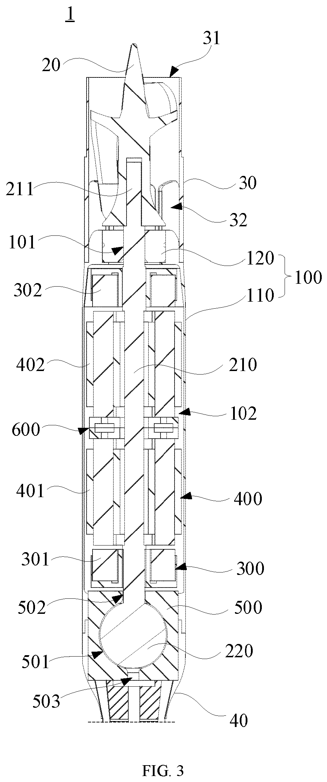

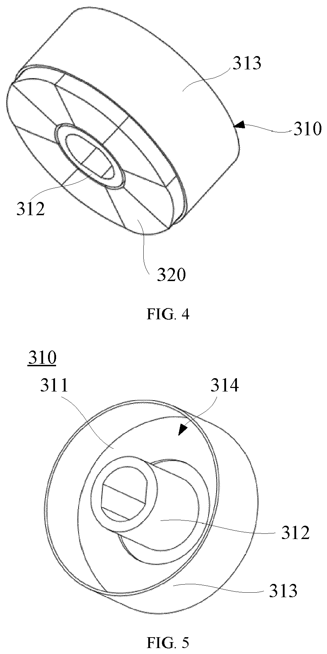

Referring to FIGS. 3 and 4, the rotor 300 is fixedly connected to the shaft portion 210. Specifically, the rotor 300 includes a flywheel 310 and a magnet 320. The flywheel 310 is fixedly connected to the shaft portion 210, and the magnet 320 is fixedly connected to the flywheel 310. In some embodiments, the magnet 320 is an annular Halbach array magnet. In the illustrated embodiment, the rotor 300 is located in the inner cavity 102, and the rotor 300 is located between the through hole 101 and the first shaft sleeve 500, that is, the rotor 300 is located between the second shaft sleeve 120 and the first shaft sleeve 500. The rotor 300 can rotate relative to the housing assembly 100 and can rotate the rotating shaft 200.

Referring to FIG. 5 together, the flywheel 310 includes a disc-shaped portion 311, an inner tube 312, and an outer tube 313. Both the inner tube 312 and the outer tube 313 are of circular tubular structures, and the disc-shaped portion 311 is of an annular disc structure. An end of the inner tube 312 and an end of the outer tube 313 are both fixedly connected to the disc-shaped portion 311. The inner tube 312 and the outer tube 313 are located on the same side of the disc-shaped portion 311 and are coaxially arranged. An inner diameter of the outer tube 313 is greater than an outer diameter of the inner tube 312. The inner tube 312 is at least partially accommodated in the outer tube 313. An annular cavity 314 accommodating the magnet 320 is formed between the outer tube 313 and the inner tube 312. A shape of the annular cavity 314 is adapted to a shape of the magnet 320, so as to facilitate mounting and positioning the magnet 320. Such arrangement enables the flywheel 310 to limit the magnet 320, which not only facilitates the mounting of the magnet 320, but also enables the magnet 320 and the flywheel 310 to be combined more stably.

It should be noted that the flywheel 310 is not limited to the above structure. In some embodiments, the flywheel 310 does not have the outer tube 313. In some embodiments, the flywheel 310 does not have the outer tube 313 and the inner tube 312. In this case, the shaft portion 210 fixedly extends through a center of the disc-shaped portion 311. Compared to the flywheel 310 having only the disc-shaped portion 311, the arrangement of the inner tube 312 enables the flywheel 310 to be more stably connected to the shaft portion 210. The shaft portion 210 may be fixedly connected to the disc-shaped portion 311 in various manners such as welding or adhering. Alternatively, the shaft portion 210 may be fixedly connected to the disc-shaped portion 311 by keeping the shaft portion 210 relatively static through a limiting structure. Alternatively, the shaft portion 210 may be fixedly connected to the flywheel 310 by synchronously rotating the shaft portion 210 and the flywheel 310 by having a plane on a contact surface between the shaft portion 210 and the inner tube 312. It should be understood that, in some embodiments, the flywheel 310 may be omitted, and in this case, the magnet 320 may be directly fixed to the shaft portion 210.

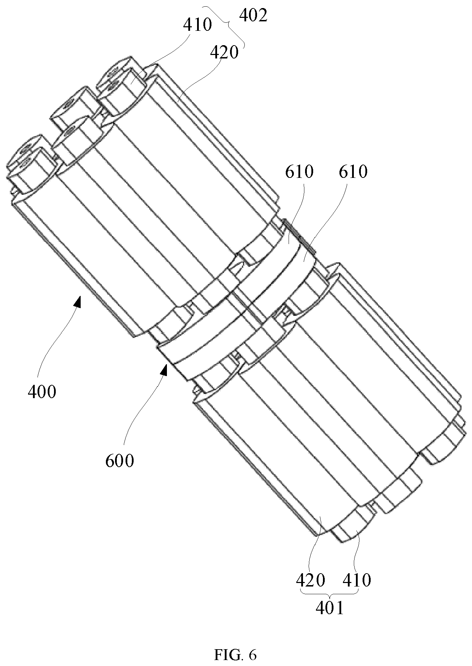

Referring to FIG. 3 and FIG. 6, the stator 400 and the rotor 300 are arranged along the axis of the shaft portion 210. The stator 400 is located between the through hole 101 and the first shaft sleeve 500, that is, the stator 400 is located between the first shaft sleeve 500 and the second shaft sleeve 120. The stator 400 can drive the rotor 300 to rotate. Specifically, the stator 400 can generate a rotating magnetic field that drives the magnet 320 to rotate. By arranging the rotor 300 and the stator 400 along the axis of the shaft portion 210, an overall diameter of the driving device 10 can be reduced. In the illustrated embodiment, the stator 400 is fixedly mounted to the housing assembly 100. The stator 400 is specifically located in the inner cavity 102. The shaft portion 210 rotatably extends through the stator 400.

In the illustrated embodiment, the rotor 300 includes a first rotor unit 301 and a second rotor unit 302 that are arranged along the axis of the shaft portion 210. Specifically, two flywheels 310 are provided, and two magnets 320 are correspondingly provided. One of the flywheels 310 and one of the magnets 320 cooperatively constitute the first rotor unit 301, and the other of the flywheels 310 and the other of the magnets 320 cooperatively constitute the second rotor unit 302. The first rotor unit 301 and the second rotor unit 302 are opposite to each other. The stator 400 is located between the first rotor unit 301 and the second rotor unit 302. The stator 400 includes a first stator unit 401 and a second stator unit 402 arranged along the axis of the shaft portion 210. The first stator unit 401 can drive the first rotor unit 301 to rotate, and the second stator unit 402 can drive the second rotor unit 302 to rotate. Specifically, the first stator unit 401 can generate a rotating magnetic field that drives the first rotor unit 301 to rotate, and the second stator unit 402 can generate a rotating magnetic field that drives the second rotor unit 302 to rotate. Both the first stator unit 401 and the second stator unit 402 are fixedly received in the inner cavity 102 of the housing assembly 100. The shaft portion 210 rotatably extends through the first stator unit 401 and the second stator unit 402. The first stator unit 401 and the second stator unit 402 are both located between the first rotor unit 301 and the second rotor unit 302. In the illustrated embodiment, the first rotor unit 301, the first stator unit 401, the second stator unit 402, and the second rotor unit 302 are arranged sequentially along the direction of the axis.

Specifically, the first stator unit 401 and the second stator unit 402 each includes a magnetic core 410 and a coil 420 wound around the magnetic core 410. The magnetic core 410 has a substantially columnar structure, that is, the magnetic core 410 does not have a head portion (i.e., a pole shoe) having a large width. Compared with the magnetic core having the pole shoe, the magnetic core 410 having the columnar structure can reduce the magnetic loss and increase the magnetic coupling density between the magnetic core 410 and the magnet 320, to increase the torque of the stator 400 to the magnet 320 (under the same current conditions). In addition, the magnetic core 410 without the head portion can also greatly reduce the problems of local magnetic short circuit and motor power reduction caused by the contact between adjacent magnetic cores 410.

Specifically, an extending direction of the magnetic core 410 coincides with the axial direction of the outer housing 110 or the axial direction of the shaft portion 210. The first stator unit 401 and the second stator unit 402 each includes a plurality of magnetic cores 410. The plurality of magnetic cores 410 of the first stator unit 401 and the second stator unit 402 are respectively arranged for one circle around the axis of the shaft portion 210. Each magnetic core 410 corresponds to one coil 420.

Specifically, the driving device 10 further includes a magnetic conduction member 600 fixedly connected to the housing assembly 100. The magnetic core 410 of the first stator unit 401 and the magnetic core 410 of the second stator unit 402 are both fixedly connected to the magnetic conduction member 600. In some embodiments, the magnetic conduction member 600 is engaged into an inner sidewall of the outer housing 110. The shaft portion 210 rotatably extends through the magnetic conduction member 600. The magnetic conduction member 600 serves to close a magnetic circuit, so as to promote and increase generation of the magnetic flux and improve a coupling capability. Therefore, the magnetic conduction member 600 can function to close the magnetic circuit between the first stator unit 401 and the first rotor unit 301, and to close the magnetic circuit between the second stator unit 402 and the second rotor unit 302, so as to increase the magnetic flux. Therefore, the arrangement of the magnetic conduction member 600 helps to reduce the overall diameter of the driving device 10. In addition, both the magnetic core 410 of the first stator unit 401 and the second magnetic core 410 of the second stator unit 402 are fixedly connected to the magnetic conduction member 600, so that the first stator unit 401 and the second stator unit 402 can be positioned and mounted by directly fixing the magnetic conduction member 600 to the housing assembly 100, and the assembling difficulty of the first stator unit 401 and the second stator unit 402 is reduced. At the same time, the magnetic conduction member 600 configured as above can also reduce arrangements of positioning structures in the housing assembly 100, thereby simplifying the structure of the housing assembly 100 and simplifying the assembling process of the entire driving device 10.

Specifically, the magnetic conduction member 600 includes two magnetic conduction plates 610 that are stacked. One of the magnetic conduction plates 610 is fixedly connected to the magnetic core 410 of the first stator unit 401, and the other of the magnetic conduction plates 610 is fixedly connected to the magnetic core 410 of the second stator unit 402. The shaft portion 210 rotatably extends through the two magnetic conduction plates 610. Optionally, the two magnetic conduction plates 610 are separated prior to assembly. Since the magnetic conduction member 600 is configured as the two magnetic conduction plates 610 that are separated prior to assembly, when assembling the driving device 10, the magnetic core 410 of the first stator unit 401 can be first fixedly connected to one of the magnetic conduction plates 610, the magnetic core 410 of the second stator unit 402 can be fixedly connected to the other of the magnetic conduction plates 610, and then the two magnetic conduction plates 610 can be stacked. In this way, the first stator unit 401 and the second stator unit 402 can be easily assembled to the two magnetic conduction plates 610, respectively, and the first stator unit 401 and the second stator unit 402 can be assembled more easily.

Specifically, the two magnetic conduction plates 600 are fixedly connected, so that the first stator unit 401, the second stator unit 402, and the magnetic conduction member 600 are integrally formed and assembled into the housing assembly 100, and the assembling of the stator 700 is easier. For example, the two magnetic conduction plates 610 may be connected together by gluing or welding. It should be understood that in other embodiments, the two magnetic conduction plates 610 are not fixedly connected, but are in contact with each other.

It should be noted that the magnetic conduction member 600 is not limited to the combination of the two magnetic conduction plates 610 that are separated. The magnetic conduction member 600 may be a plate-like structure, that is, the magnetic conduction member 600 is one magnetic conduction plate 610. In this case, the first stator unit 401 and the second stator unit 402 share one magnetic conduction plate 610.

Specifically, the magnetic conduction plate 610 is made of silicon steel, and the magnetic core 410 is made of silicon steel.

It should be understood that the structures of the rotor 300 and the stator 400 are not limited to the above structures. In some embodiments, the driving device 10 includes a first rotor unit 301, a second rotor unit 302, and a stator 400, but the stator 400 has only one stator unit. The stator unit is located between the first rotor unit 301 and the second rotor unit 302, and the stator unit can drive the first rotor unit 301 and the second rotor unit 302 to rotate simultaneously. In this case, the magnetic conduction member 600 is omitted. Alternatively, in some embodiments, the rotor 300 has only one rotor unit and the stator 400 has only one stator unit. In this case, the rotor unit is located between the first shaft sleeve 500 and the stator unit, or the rotor unit is located between the stator unit and the second shaft sleeve 120. The number of the stator units of the stator 400 and the number of the rotor units of the rotor 300 may be adjusted as required.

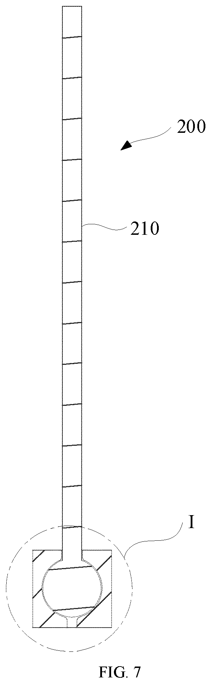

Referring to FIGS. 3 and 7, the first shaft sleeve 500 is mounted to the housing assembly 100. Specifically, the first shaft sleeve 500 is received in the inner cavity 102 of the housing assembly 100. The first shaft sleeve 500 is fixedly connected to the outer housing 110. The rotor 300, the stator 400, and the first shaft sleeve 500 are spaced apart along the axial direction of the outer housing 110. In the illustrated embodiment, the first shaft sleeve 500 is located on a side of the first rotor unit 301 away from the stator 400.

In some embodiments, there is a certain gap between the first shaft sleeve 500 and the first rotor unit 301, so as to avoid wear caused by the contact between the first shaft sleeve 500 and the first rotor unit 301. However, the gap between the first shaft sleeve 500 and the first rotor unit 301 should not be too large, so as to prevent the length of the rotating shaft 200 from being too long and improve the stress of the rotating shaft 200.

The first shaft sleeve 500 is provided with a receiving cavity 501 and a shaft hole 502. The receiving cavity 501 is adapted to the spherical head portion 220. The shaft hole 502 is in communication with the receiving cavity 501. The spherical head portion 220 is rotatably received in the receiving cavity 501. The shaft portion 210 rotatably extends through the shaft hole 502. A diameter of the shaft hole 502 is less than the diameter of the spherical head portion 220, so as to limit the spherical head portion 220 in the receiving cavity 501. Specifically, the receiving cavity 501 is substantially spherical, or a cavity wall of the receiving cavity 501 encloses a spherical structure.

Since the end of the shaft portion 210 away from the spherical head portion 220 rotatably extends through the through hole 101 of the housing assembly 100, a hole wall of the through hole 101 limits a swinging range of the end of the shaft portion 210 away from the spherical head portion 220 in the radial direction. In addition, the spherical head portion 220 fixedly connected to the end of the shaft portion 210 is rotatably received in the receiving cavity 501 adapted to the spherical head portion 220, and the diameter of the spherical head portion 220 is greater than the diameter of the shaft hole 502, so that the spherical head portion 220 is limited in the receiving cavity 501. As such, the first shaft sleeve 500 limits the swinging range of the end of the shaft portion 210 adjacent to the spherical head portion 220 in the radial direction, and simultaneously limits a moving range of the spherical head portion 220 along the axis of the shaft portion 210. That is, an axial limiting and a radial limiting of the rotating shaft 200 are achieved, and a thrust member is not required to be additionally provided, which simplify the structure of the driving device 10, and helps to reduce the assembling difficulty of the driving device 10 and the blood pump 1.

Meanwhile, since the rotating shaft 200 and the first shaft sleeve 500 realize the swinging range of the end of the rotating shaft 200 adjacent to the spherical head portion 220 in the radial direction and the moving range of the rotating shaft 200 along the axis of the shaft portion 210 through the cooperation of the spherical head portion 220 and the receiving cavity 501 adapted to the spherical head portion 220, which is conducive to reducing the friction between the spherical head portion 220 and the first shaft sleeve 500, thereby reducing the wear of the rotating shaft 200.

Specifically, the receiving cavity 501 being adapted to the spherical head portion 220 means that a shape of the cavity wall of the receiving cavity 501 is the same as a shape of the spherical head portion 220, and the diameter of the spherical head portion 220 is slightly less than the diameter of the receiving cavity 501, so that the spherical head portion 220 not only can rotate in the receiving cavity 501, but also the cavity wall of the receiving cavity 501 can support the spherical head portion 220 and limit the spherical head portion 220 in each direction. In addition, surfaces of the cavity wall of the receiving cavity 501 and the spherical head portion 220 are smooth arc surfaces, which can reduce the wear of the receiving cavity 501 and the spherical head portion 220.

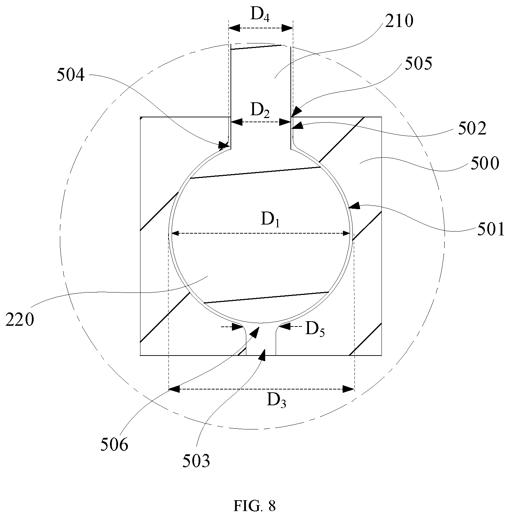

Referring to FIG. 7 and FIG. 8, the shaft hole 502 has a first opening 504 and a second opening 505. The shaft portion 210 extends through the first opening 504 and the second opening 505. The first opening 504 is in communication with the receiving cavity 501, and the second opening 505 is away from the receiving cavity 501. Specifically, an edge of at least one of the first opening 504 and the second opening 505 is provided with a rounded corner to prevent the shaft portion 210 or the spherical head portion 220 from being scratched and worn by the opening of the shaft hole 502 having a sharp corner.

Specifically, the first shaft sleeve 500 is further provided with a flushing liquid hole 503 through which the flushing liquid flows. The flushing liquid hole 503 is in fluid communication with the receiving cavity 501. A diameter of the flushing liquid hole 503 is less than the diameter of the spherical head portion 220, so that the spherical head portion 220 is limited in the receiving cavity 501. Specifically, the flushing liquid hole 503 can be in communication with a flushing pipeline in the catheter 40 and thus can be in fluid communication with the cleaning pipeline, such that the flushing fluid can enter the receiving cavity 501 through the flushing liquid hole 503.

Specifically, the flushing liquid hole 503 has a third opening 506 in communication with the receiving cavity 501. A central axis of the third opening 506 passes through a center of the receiving cavity 501 or a center of a sphere where the receiving cavity 501 is located, and the central axis of the third opening 506 coincides with a central axis of the shaft hole 502. In this way, the flushing liquid can well enter between the cavity wall of the receiving cavity 501 and the spherical head portion 220, which not only serves as a lubrication and reduces a friction coefficient between the spherical head portion 220 and the cavity wall of the receiving cavity 501, thereby reducing the wear of the spherical head portion 220 and the first shaft sleeve 500, but also enables the flushing liquid entering the receiving cavity 501 from the flushing liquid hole 503 to provide a hydraulic suspension support for the spherical head portion 220. An opening of the flushing liquid hole 503 away from the third opening 506 is in communication with the flushing pipeline in the catheter 40. More specifically, the central axis of the flushing liquid hole 503 coincides with the central axis of the receiving cavity 501, so that the flushing liquid hole 503 is a straight hole, to reduce an energy consumption of the flushing liquid in the flushing liquid hole 503.

In some embodiments, the diameter of the spherical head portion 220 is defined as D₁, and the diameter of the end of the shaft portion 210 adjacent to the spherical head portion 220 is defined as D₂. In an embodiment, the diameter D₁ of the spherical head portion 220 is greater than or equal to twice the diameter D₂ of the end of the shaft portion 210 adjacent to the spherical head portion 220, i.e., D₁ ≥ 2*D₂, so that the spherical head portion 220 is large enough, i.e., the diameter of the spherical head portion 220 is large enough, such that when the spherical head portion 220 is in contact with the cavity wall of the receiving cavity 501, the spherical head portion 220 has a large contact area with the cavity wall of the receiving cavity 501, which reduces the wear between the spherical head portion 220 and the cavity wall of the receiving cavity 501. Specifically, the diameter D₁ of the spherical head portion 220 need not be excessively large, so as to avoid the overall diameter of the blood pump 1 being too large.

In an embodiment, the diameter of the receiving cavity 501 is defined as D₃, and a ratio of the diameter D₃ of the receiving cavity 501 to the diameter D₁ of the spherical head portion 220 is in a range of 1.005 to 1.015, i.e., D₃: D₁ is in a range of 1.005 to 1.015. The diameter D₃ of the receiving cavity 501 to the diameter D₁ of the spherical head portion 220 are controlled to the above ratio, which not only enables the spherical head portion 220 to have a certain movement space in the receiving cavity 501 to ensure that the receiving cavity 501 has a proper space to be filled with the flushing liquid and the flushing liquid can be smoothly circulated, but also can limit an axial movement and a radial movement of the rotating shaft 200 within a reasonable range to ensure stable rotation of the rotating shaft 200. At the same time, wear of the spherical head portion 220 and the cavity wall of the receiving cavity 501 is small. If a difference between the diameter of the spherical head portion 220 and the diameter of the receiving cavity 501 is too small, the circulation of the flushing liquid may be poor, and the hydraulic suspension support effect of the flushing liquid for the spherical head portion 220 may be poor. If the difference between the diameter of the spherical head portion 220 and the diameter of the cavity wall of the receiving cavity 501 is too large, the contact surface between the spherical head portion 220 and the cavity wall of the receiving cavity 501 may be too small, which may increase the wear of the spherical head portion 220 and the cavity wall of the receiving cavity 501, and the spherical head portion 220 moves greatly in the receiving cavity 501, which causes the driving device 10 to operate unstably.

In an embodiment, the diameter of the shaft hole 502 is defined as D₄, and a ratio of the diameter D₄ of the shaft hole 502 to the diameter D₂ of the end of the shaft portion 210 adjacent to the spherical head portion 220 is in a range of 1.15 to 1.35, i.e., D₄: D₂ is in a range of 1.15 to 1.35. Due to the problem of mounting or machining accuracy, when the first shaft sleeve 500 and the hole wall of the through hole 101 limit the shaft portion 210, it is difficult to ensure that the axis of the shaft portion 210 coincides with or is completely parallel to the axial direction of the outer housing 110. Therefore, a clearance space needs to be reserved between the shaft hole 502 and the shaft portion 210 to provide tolerance for the shaft portion 210. If a difference between the diameter of the shaft hole 502 and the diameter of the shaft portion 210 is relatively small, the shaft portion 210 may be abutted against or stuck by the hole wall of the shaft hole 502 during rotation, which causes the rotating shaft 200 to rotate unsmoothly, and causes the shaft portion 210 and the hole wall of the shaft hole 502 to be severely worn at the abutted position. Meanwhile, the small difference between the diameter of the shaft hole 502 and the diameter of the shaft portion 210 is not conducive to the flushing liquid flowing from the receiving cavity 501 into the inner cavity 102 of the housing assembly 100, which causes the flushing liquid to flow unsmoothly. If the diameter of the shaft hole 502 and the diameter of the shaft portion 210 are relatively large, the shaft portion 210 may swing too much, and the contact area between the spherical head portion 220 and the cavity wall of the receiving cavity 501 may be reduced, especially when the spherical head portion 220 is in contact with the cavity wall of the receiving cavity 501 adjacent to the shaft hole 502, and the spherical head portion 220 may be worn seriously.

In an embodiment, a difference between the diameter D₃ of the receiving cavity 501 and the diameter D₁ of the spherical head portion 220 is defined as d1. A difference between the diameter D₄ of the shaft hole 502 and the diameter D₂ of a portion of the shaft portion 210 adjacent to the spherical head portion 220 is defined as d2. d2 is greater than d1, so that a tolerance space can be reserved for the shaft portion 210.

In an embodiment, a diameter of the third opening 506 of the flushing liquid hole 503 is defined as D₅. The diameter D₅ of the third opening 506 of the flushing liquid hole 503 is 1/9 to 1/3 of the diameter D₁ of the spherical head portion 220. If the diameter of the third opening 506 of the flushing liquid hole 503 is too large, the contact surface between the spherical head portion 220 and the cavity wall of the receiving cavity 501 will be reduced (resulting in a large pressure per unit area), and the wear of the spherical head portion 220 by the cavity wall of the receiving cavity 501 will be increased. If the diameter of the third opening 506 is too small, the amount of the flushing liquid entering the receiving cavity 501 from the flushing liquid hole 503 will be affected. However, the flushing liquid entering the flushing liquid hole 503, on the one hand, gives an impulse to the spherical head portion 220, and at the same time, enters between the spherical head portion 220 and the cavity wall of the receiving cavity 501 to servers as a lubrication, so as to reduce the friction coefficient between the spherical head portion 220 and the cavity wall of the receiving cavity 501. Therefore, the amount of the flushing liquid entering the receiving cavity 501 should not be too small.

In addition, an edge of the third opening 506 of the flushing liquid hole 503 is provided with a rounded corner to prevent the spherical head portion 220 from being scratched and worn.

In some embodiments, the first shaft sleeve 500 includes a first fixing portion 510 and a second fixing portion 520. The first fixing portion 510 and the second fixing portion 520 cooperatively enclose the receiving cavity 501, so that the spherical head portion 220 fits into the receiving cavity 501. The first fixing portion 510 and the second fixing portion 520 may be fixed together by adhering, welding, engagement or other manners. The first fixing portion 510 and the second fixing portion 520 may only be in contact with each other without physical fixed connection. Even the first fixing portion 510 and the second fixing portion 520 may face each other with a gap therebetween.

In an embodiment, referring to FIG. 3, FIG. 9 and FIG. 10, a side of the first fixing portion 510 is recessed to form a first half-hole 511 and a first hemispherical groove 512 that are in communication with each other. A side of the second fixing portion 520 is recessed to form a second half-hole 521 and a second hemispherical groove 522 that are in communication with each other. A concave side of the first fixing portion 510 and a concave side of the second fixing portion 520 face each other. The first half-hole 511 and the second half-hole 521 cooperatively form the shaft hole 502. The first hemispherical groove 512 and the second hemispherical groove 522 cooperatively form the receiving cavity 501. In this way, the concave side of the first fixing portion 510 and the concave side of the second fixing portion 520 face each other, so that the assembling is convenient, and the spherical head portion 220 is wrapped from a left side and a right side of the spherical head portion 220 without considering the characteristics of the shaft portion 210. For example, if the end of the shaft portion 210 adjacent to the spherical head portion 220 has sections with different diameters, the diameter of the shaft hole 502 does not have to be greater than the maximum diameter of the shaft portion 210 to allow the shaft portion 210 to fit through the shaft hole 502.

Specifically, the concave side of the first fixing portion 510 is further concave to form a first semi-liquid groove 513, the concave side of the second fixing portion 520 is further concave to form a second semi-liquid groove 523. The first semi-liquid groove 513 and the second semi-liquid groove 523 cooperatively form the flushing liquid hole 503.

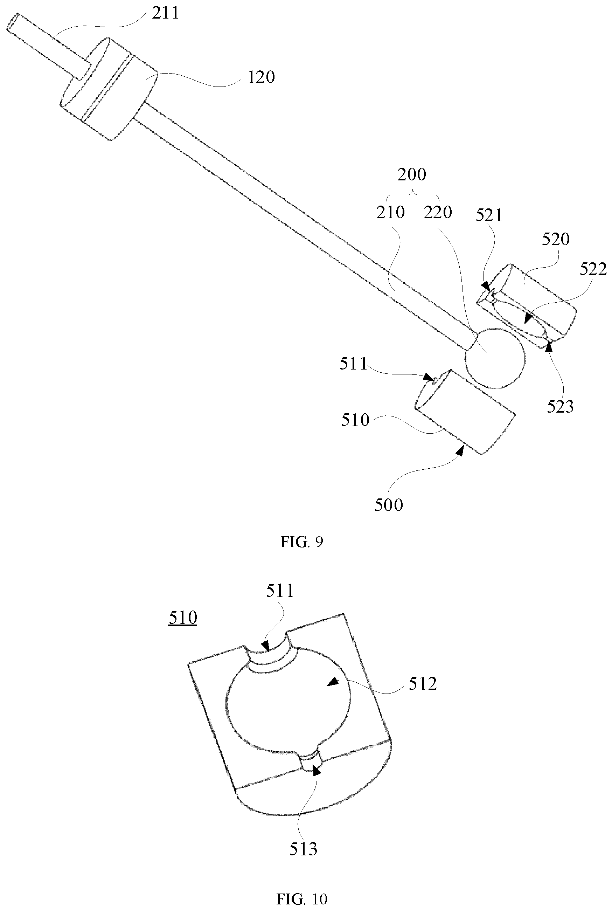

In the embodiment shown in FIG. 9, the first shaft sleeve 500 is divided into the first fixing portion 510 and the second fixing portion 520 along a plane passing through the axis of the shaft portion 210, and the first fixing portion 510 and the second fixing portion 520 are symmetrically distributed with respect to the plane passing through the axis of the shaft portion 210. It should be understood that, in other embodiments, the first shaft sleeve 500 is divided into the first fixing portion 510 and the second fixing portion 520 along a plane at a certain angle with the axis of the shaft portion 210, and the first fixing portion 510 and the second fixing portion 520 are not necessarily the same in size and shape.

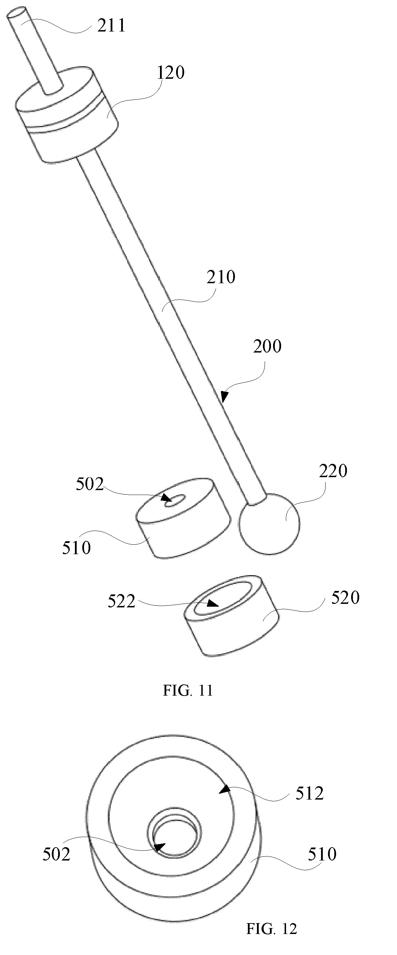

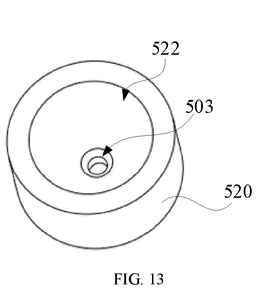

In another embodiment, referring to FIG. 11 to FIG. 13, the first fixing portion 510 is provided with a first hemispherical groove 512 and a shaft hole 502 in communication with the first hemispherical groove 512; the second fixing portion 520 has a second hemispherical groove 522. The first hemispherical groove 512 and the second hemispherical groove 522 cooperatively form the receiving cavity 501. In this case, a splicing surface of the first fixing portion 510 and the second fixing portion 520 is arranged around the axis of the shaft portion 210. An upper surface of the cavity wall of the receiving cavity 501 is the first hemispherical groove 512 of the first fixing portion 510, and a lower surface of the cavity wall of the receiving cavity 501 is the second hemispherical groove 522 of the second fixing portion 520. The upper surface and the lower surface of the cavity wall of the receiving cavity 501 are separately provided without a splicing gap, and a semicircular shape is more complete. Therefore, the corresponding contact surface of the spherical head portion 220 in the axial direction of the shaft portion 210 is a complete smooth semicircle, which is conducive to reducing the wear between the spherical head portion 220 and the cavity wall of the receiving cavity 501, and can reduce the assembling process requirements of the first fixing portion 510 and the second fixing portion 520.

In the embodiment shown in FIG. 11, the first shaft sleeve 500 is divided into the first fixing portion 510 and the second fixing portion 520 arranged vertically along a plane substantially perpendicular to the axis of the shaft portion 210.

Referring to FIG. 2 and FIG. 3 again, one end of the rotating shaft 200 is the spherical head portion 220, which is rotatably supported on the first shaft sleeve 500, and the other end of the rotating shaft 200 is the end of the shaft portion 210 away from the spherical head portion 220, which is rotatably supported on the second shaft sleeve 120. Therefore, both ends of the rotating shaft 200 are supported, and the force on the rotating shaft 200 is stable, so that the rotating shaft 200 and the rotor 300 operate smoothly.

Specifically, at least one of the first shaft sleeve 500 and the second shaft sleeve 120 is made of a ceramic material. Compared with metal materials, ceramics have higher processing precision, higher biocompatibility and mechanical strength, and better wear resistance and corrosion resistance. In some embodiments, at least one of the first shaft sleeve 500 and the second shaft sleeve 120 has a shaft sleeve body and a diamond coating provided on a surface of the shaft sleeve body, so that the surfaces of the first shaft sleeve 500 and the second shaft sleeve 120 are smooth and wear resistance thereof is improved. In this case, a material of the shaft sleeve body may have a certain rigidity, such as metal, ceramic, etc.

Specifically, referring to FIG. 8, a roughness of at least one of the hole wall of the through hole 101, the surface of the shaft portion 210, the surface of the spherical head portion 220, the cavity wall of the receiving cavity 501, and the hole wall of the shaft hole 502 is less than or equal to 0.1 micron, so as to effectively reduce the friction between the shaft portion 210 and the hole wall of the through hole 101, the friction between the shaft portion 210 and the hole wall of the shaft hole 502, and the friction between the spherical portion 220 and the cavity wall of the receiving cavity 501.

The foregoing embodiments are merely intended to describe the technical solutions of the present application, but not to limit the present application. Although the present application is described in detail with reference to the foregoing embodiments, those of ordinary skill in the art should understand that they may still make modifications to the technical solutions described in the foregoing embodiments or make equivalent replacements to some technical features thereof, without departing from the spirit and scope of the technical solutions of the embodiments of the present application, all of which fall within the protection scope of the present application.

## Claims

1. A driving device, comprising:
a housing assembly provided with a through hole;
a rotating shaft comprising a shaft portion and a spherical head portion fixedly connected to an end of the shaft portion, an end of the shaft portion away from the spherical head portion rotatably extending through the through hole; and
a first shaft sleeve mounted to the housing assembly, wherein the first shaft sleeve is provided with a receiving cavity and a shaft hole, the receiving cavity is adapted to the spherical head portion, and the shaft hole is in communication with the receiving cavity;
wherein the spherical head portion is rotatably received in the receiving cavity; the shaft portion rotatably extends through the shaft hole; and a diameter of the shaft hole is less than a diameter of the spherical head portion, so as to limit the spherical head portion in the receiving cavity.

2. The driving device according to claim 1, wherein the diameter of the spherical head portion is greater than or equal to twice a diameter of an end of the shaft portion adjacent to the spherical head portion.

3. The driving device according to claim 1, wherein the receiving cavity is spherical, and a ratio of a diameter of the receiving cavity to the diameter of the spherical head portion is in a range of 1.005 to 1.015.

4. The driving device according to claim 1, wherein the receiving cavity is spherical, a difference between a diameter of the receiving cavity and the diameter of the spherical head portion is defined as d1, a difference between the diameter of the shaft hole and a diameter of an end of the shaft portion adjacent to the spherical head portion is defined as d2, and d2 is greater than d1.

5. The driving device according to claim 1, wherein a ratio of the diameter of the shaft hole to a diameter of an end of the shaft portion adjacent to the spherical head portion is in a range of 1.15 to 1.35.

6. The driving device according to claim 1, wherein the first shaft sleeve comprises a first fixing portion and a second fixing portion;
a side of the first fixing portion is recessed to form a first half-hole and a first hemispherical groove that are in communication with each other, a side of second fixing portion is recessed to form a second half-hole and a second hemispherical groove that are in communication with each other; a concave side of the first fixing portion and a concave side of the second fixing portion face each other; the first half-hole and the second half-hole cooperatively form the shaft hole; and the first hemispherical groove and the second hemispherical groove cooperatively form the receiving cavity;
or, the first fixing portion is provided with a first hemispherical groove and a shaft hole in communication with the first hemispherical groove, the second fixing portion has a second hemispherical groove; and the first hemispherical groove and the second hemispherical groove cooperatively form the receiving cavity.

7. The driving device according to claim 1, wherein the first shaft sleeve is further provided with a flushing liquid hole through which flushing liquid flows; the flushing liquid hole is in communication with the receiving cavity; and a central axis of the flushing liquid hole coincides with a central axis of the receiving cavity.

8. The driving device according to claim 1, wherein the housing assembly comprises an outer housing and a second shaft sleeve; the second shaft sleeve and the first shaft sleeve are both fixed in the outer housing, and the through hole is formed on the second shaft sleeve.

9. The driving device according to claim 8, wherein one end of the rotating shaft is the spherical head portion, the spherical head portion is rotatably supported on the first shaft sleeve, another end of the rotating shaft is the end of the shaft portion away from the spherical head portion and is rotatably supported on the second shaft sleeve.

10. The driving device according to claim 1, wherein the shaft hole has a first opening and a second opening; the first opening is in communication with the receiving cavity, the second opening is away from the receiving cavity; an edge of at least one of the first opening and the second opening is provided with a rounded corner; and the shaft portion extends through the first opening and the second opening.

11. The driving device according to any one of claims 1 to 10, further comprising a rotor and a stator arranged along an axis of the shaft portion, wherein the rotor and the stator are located between the first shaft sleeve and the through hole, the rotor is fixedly connected to the shaft portion, and the stator is capable of driving the rotor to rotate.

12. The driving device according to claim 11, wherein the rotor comprises a flywheel and a magnet; the flywheel is fixedly connected to the shaft portion; the magnet is fixedly connected to the flywheel; the stator is capable of generating a rotating magnetic field that drives the magnet to rotate; the flywheel comprises a disc-shaped portion, an inner tube, and an outer tube; an end of the inner tube and an end of the outer tube are both fixedly connected to the disc-shaped portion; the inner tube and the outer tube are located on a same side of the disc-shaped portion and are coaxially arranged; an inner diameter of the outer tube is greater than an outer diameter of the inner tube; the inner tube is at least partially accommodated in the outer tube; and an annular cavity accommodating the magnet is formed between the outer tube and the inner tube.

13. The driving device according to claim 11, wherein the rotor comprises a first rotor unit and a second rotor unit that are arranged along the axis of the shaft portion; the stator comprises a first stator unit and a second stator unit that are arranged along the axis of the shaft portion; the first stator unit and the second stator unit are both located between the first rotor unit and the second rotor unit; the first stator unit is capable of driving the first rotor unit to rotate; and the second stator unit is capable of driving the second rotor unit to rotate; and the first stator unit and the second stator unit each comprises a magnetic core;
the driving device further comprises a magnetic conduction member fixedly connected to the housing assembly; the magnetic core of the first stator unit and the magnetic core of the second stator unit are both fixedly connected to the magnetic conduction member; and the shaft portion rotatably extends through the first stator unit, the second stator unit, and the magnetic conduction member.

14. The driving device according to claim 1, wherein the first shaft sleeve is further provided with a flushing liquid hole through which a flushing liquid flows; the flushing liquid hole is in fluid communication with the receiving cavity; and a diameter of the flushing liquid hole is less than the diameter of the spherical head portion.

15. The driving device according to claim 14, wherein the flushing liquid hole has a third opening in communication with the receiving cavity; the receiving cavity is spherical; a central axis of the third opening passes through a center of a sphere where the receiving cavity is located; and the central axis of the third opening coincides with a central axis of the shaft hole.

16. The driving device according to claim 15, wherein a diameter of the third opening of the flushing liquid hole is 1/9 to 1/3 of the diameter of the spherical head portion.

17. The driving device according to claim 1, wherein a central axis of the shaft portion passes through a center of a sphere of the spherical head portion.

18. The driving device according to claim 1, wherein the spherical head portion comprises a ball body and a diamond coating provided on a surface of the ball body, or the spherical head portion is made of metal or ceramic.

19. A blood pump comprising an impeller and a driving device, the driving device comprising:
a housing assembly provided with a through hole;
a rotating shaft comprising a shaft portion and a spherical head portion fixedly connected to an end of the shaft portion, an end of the shaft portion away from the spherical head portion rotatably extending through the through hole; and
a first shaft sleeve mounted to the housing assembly, wherein the first shaft sleeve is provided with a receiving cavity and a shaft hole; the receiving cavity is adapted to the spherical head portion; the shaft hole is in communication with the receiving cavity;
wherein the spherical head portion is rotatably received in the receiving cavity; the shaft portion rotatably extends through the shaft hole; a diameter of the shaft hole is less than a diameter of the spherical head portion, so as to limit the spherical head portion in the receiving cavity; and the end of the shaft portion away from the spherical head portion is connected to the impeller.
